# EUROPEAN PATENT SPECIFICATION

(11) **EP 2 310 448 B1**
(45) Date of publication and mention of the grant of the patent: **19.10.2016**
(21) Application number: 09800060.7
(22) Date of filing: 22.07.2009
(51) Int. Cl.: C08L 5/04, C08L 5/08, C08B 37/08

(54) **THREE-DIMENSIONAL NANOCOMPOSITE MATERIALS CONSISTING OF A POLYSACCHARIDIC MATRIX AND METALLIC NANOPARTICLES, PREPARATION AND USE THEREOF**
DREIDIMENSIONALE NANOKOMPOSITMATERIALIEN AUS EINER POLYSACCHARIDMATRIX UND METALLISCHEN NANOPARTIKELN, HERSTELLUNG UND VERWENDUNG
MATÉRIAUX NANOCOMPOSITES TRIDIMENSIONNELS CONSISTANT EN UNE MATRICE POLYSACCHARIDE ET DES NANOPARTICULES MÉTALLIQUES, LEUR PRÉPARATION ET LEUR UTILISATION

(30) Priority: 23.07.2008 IT PD20080220
(43) Date of publication of application: 20.04.2011
(73) Proprietor: Universita Degli Studi di Trieste, 34127 Trieste (IT)
(72) Inventor: DONATI, Ivan, I-33039 Sedegliano (IT); MARSICH, Eleonora, I-34148 Trieste (IT); TRAVAN, Andrea, I-34100 Trieste (IT); PAOLETTI, Sergio, I-34136 Trieste (IT)
(74) Representative: Gervasi, Gemma
(86) International application number: PCT/EP2009/059432
(87) International publication number: WO 2010/010123

(56) References cited:
- WO-A-2007/135116
- US-A1- 2008 147 019

## Description

### Field of the invention

The present invention relates to three-dimensional nanocomposite materials comprising a complex polymeric matrix consisting of a polysaccharidic composition of neutral or anionic polysaccharides and of branched cationic polysaccharides, in which metallic nanoparticles are uniformly dispersed and stabilized, said branched cationic polysaccharides so forming a metal-based nanocomposite. The invention further concerns the preparation and use of said three-dimensional nanocomposite materials in biomedical, pharmaceutical and food fields.

### State of the art

Natural polysaccharides have been generally recognized as biocompatible polymers; as such, they are well-studied materials employed since long time for applications in biomedical field, for example as carriers of biologically active compounds or cells for tissue engineering. Among the most used ones in both pharmaceutical and food industry, alginates and chitosan may be mentioned for their abundance, relatively low cost, high biocompatibility and ability to produce in appropriate conditions three-dimensional matrices in the form of hydrogels with high water content. However, chitosan exhibits some application limits linked to its marked dependence of its water solubility from pH, its non-miscibility in aqueous solutions with anionic polysaccharides such as alginate, with which it produces coacervates not usable for applicative purposes, such as for example in tissue engineering. Chitosan shares with alginates a further limit inasmuch as it does not carry any cell-specific signal thus lacking bioactivity. For these reasons chitosan derivatives, which can overcome the above-mentioned limits, are currently studied and developed.

In the recent years, different chitosan derivates were obtained by means of chemical modification of the polymeric chain. For these modifications, reactions involving the amino residue of the D-glucosamine units, forming the linear chitosan chain, are extensively used. In particular, introducing saccharidic units (mono- and oligo-saccharides) as the N-linked side chain, allowed to obtain water-soluble chitosan derivatives without the need of lowering pH down to acidic values, in this manner also avoiding the possible resulting problems of degradation of the polymer due to the acidity of the aqueous solutions.

In US 4,424,346 (Hall, L.D. and Yalpani, M.) the synthesis of these derivatives was described for the first time as well as the aqueous solubility thereof in a non-acidic aqueous medium. In particular, US 4,424,346 disclosed that the chitosan derivative with lactose produces rigid gels in aqueous solutions at concentrations higher than 3-5%, while it does not gel nor precipitate in salts or acids mixtures (in particular with Ca, Cr, Zn chlorides, K chromate, boric acid) and combinations thereof. Moreover, the aforesaid patent mentioned the fact that the chitosan derivatized with another oligosaccharide, that is cellobiose, does not form gels in aqueous solutions *per se,* while it forms rigid gels when mixed with alginate. This gel formation is due to the strong interaction between the positive polycation charges and the negative polyanion charges, which leads to a system coacervation, a process that otherwise is a limit in its use, for example, for microencapsulation of biologic material such as cells.

Patent Application WO2007/135116 (Paoletti S. et al.) describes methods for preparing polymeric solutions containing mixtures of anionic and cationic polysaccharides to overcome the problem of coacervation and the use thereof in biomedical field.

In addition, Patent Application WO2007/135114 (Paoletti S. et al.) describes three-dimensional structures, both hydrated or non-hydrated, and methods of preparing them from the above-mentioned polymeric mixtures of anionic and cationic polysaccharides gelled with appropriate gelling agents, useful for the purpose to encapsulate pharmacologically active molecules and cells.

Another remarkably interesting research area releated to nanotechnologies and with a great topical interest for polysaccharides is their possible use for preparing nanocomposite materials, comprising in particular metallic nanoparticles. Indeed, in order to stabilize the nanoparticles, polysaccharidic solutions, which allow to obtain nanocomposite systems wherein the metallic particles are homogeneously dispersed due to the interactions with the polymeric chains, could be profitably used. Therefore, the role of polysaccharides is related to formation and stabilization of metallic nanoparticles by expecting the possibility of exploiting their particular properties; indeed, metallic nanoparticles are known to be provided with particular optical, catalytic and antimicrobial properties. In fact, the use of metals, such as silver, gold, copper, zinc and nickel, in the field of antimicrobial materials is greatly impacting on the market, especially for treating skin wounds. Companies such as Johnson&Johnson^{©} and Convatec^{©} recently have commercialized medications based on the antibacterial properties of silver nanoparticles. Similar applications could be found by appropriate polysaccharides-based nanocomposite materials to exploit the well-known antimicrobial activity of these metals, for example, for the development of gauzes, bandages, patches. The latter products could be endowed with a broad-spectrum antimicrobial activity or high water content gels with bactericidal activity. Indeed, the need for novel therapeutic aids for treating skin or mucosa lesions, such as burns and ulcerations, is still felt. These lesions are often very resistant to the currently adopted antibiotic therapies; in addition, they also need further biological effects for tissue repair, for example enhancing cell proliferation, and/or appropriate tissue hydration. The three-dimensional hydrogel structure may be particularly advantageous especially for the latter aspect, being able to ensure an appropriate environment for cell replication without interfering with cell phenotype.

Furthermore, the possibility of obtaining three-dimensional hydrated structures is also particularly interesting for tissue-engineering applications, where combining bioactive properties typical of polysaccharides with antimicrobial activity is desirable.

In the field of tissue-engineering, a great effort is being made to produce antimicrobial coatings on biomaterials to be implanted into human body; in this case, the major risk factor is related to the possible cytotoxicity of the antimicrobial agents themselves. For example, in the orthopedic surgery field, the prosthetic joint replacement operations and osteosynthesis of unexposed fractures represent a type of clean surgery with regards to surgery infections (Tucci G. et al., Giornale Italiano di Ortopedia e Traumatologia, 2005; 31:121-129). However, implantation of biomaterials within host tissues may promote the onset and subsistence of infections even with somewhat low bacterial loads. Despite the progress in perioperative prophylaxis, bacterial and fungal infections are still very common; this is caused by time-extended risk of adhesion of these microorganisms to the orthopedic device (Zimmerli W. et al., New England J. Med., 2004; 351(16):1645-54). These data support the importance of developing alternative antimicrobial agents to be associated with new-generation biomaterials.

US 7255881 (Gillis et al.) provides a possible solution to the above-mentioned problems. In fact, the patent discloses silver-based coatings formed on various types of substrate through techniques such as chemical and physical depositions from a vapour phase ("chemical vapour deposition" CVD, "physical vapour deposition" PVD) and in a liquid phase for antimicrobial applications. Regarding the polysaccharidic substrates, on which silver is deposited, chitosan, alginate and hyaluronic acid are mentioned. It is noted that these techniques are not directed to the formation of metallic nanoparticles homogeneously dispersed within appropriate matrices, but address the formation of continuous, surface silver (nanocrystalline, polycrystalline or amorphous) layers. Furthermore, the temperature and pressure conditions required for these deposition techniques are not compatible with the stability of the polysaccharide nor of bioactive biomolecules (like peptides or proteins) that might be desirable to be part of the scaffold, nor, even more so, with tissue engineering applications involving living cells.

### Summary

It is a first object of the present invention to provide three-dimensional nanocomposite systems, where size-controlled metallic nanoparticles are homogeneously dispersed into polysaccharidic matrices, being said matrices in the gel or in the solid form, and the properties of which are particularly suitable for biologic applications in the biomedical field.

It is a further object that such a three-dimensional nanocomposite is obtainable by a simple and economically convenient chemical approach, and in particular, but not exclusively, by producing biocompatible and bioactive hydrogels and dehydrated hydrogels.

It is a further object to improve these systems by employing readily commercially available polysaccharides and without these polysaccharides being subjected to chemical manipulations, as well as without the need for complex preparative manipulations of these systems.

In order to fulfill the above-mentioned objects, the inventors developed suitable polysaccharidic systems based on at least binary compositions comprising neutral or anionic polysaccharides, preferably derived from vegetal or bacterial sources, and branched cationic polysaccharides allowing the later polysaccharides to entrap metallic nanoparticles, and being the neutral or anionic polysaccharides able to form three-dimensional solid matrices hydrated or non-hydrated (e.g. hydrogels with various forms, microspheres, scaffolds, fibrous matrices) and/or high surface/volume ratio matrices (wet or dehydrated membranes and films). In this manner the system formed by branched cationic polysaccharides, uniformly entrapping metal nanoparticles, is itself a nanocomposite.

Therefore, in a first aspect the object of the invention consists in three-dimensional nanocomposite materials comprising a polymeric matrix consisting of at least one neutral or anionic (lyotropic, thermotropic or thermo-lyotropic) polysaccharide, and a metal-based nanocomposite consisting of at least one branched cationic polysaccharide wherein metallic nanoparticles are uniformly dispersed and stabilized, where the neutral or anionic polysaccharide is gelled by means of suitable physical or chemical gelling agents, depending on the type of the neutral or anionic polysaccharide itself.

It was possible to mix solutions of metal nanoparticles-containing cationic branched polysaccharides with neutral or anionic polysaccharide solutions and working with suitable pH value and ionic strength, in order not to cause formation of coacervates. By exploiting the neutral or acidic polysaccharide ability to form ionotropic or thermotropic gels, by means of suitable gelling agents, it was then possible to obtain three-dimensional matrices, hydrated or non-hydrated, consisting of mixtures of these neutral or anionic polysaccharides and branched basic polysaccharides, entrapping these latter metallic nanoparticles uniformly dispersed and stabilized through the branched basic polysaccharide itself. Therefore, in a second aspect, it is an object of the invention a method of preparing three-dimensional nanocomposite materials comprising a polymeric matrix consisting of at least one lyotropic, thermotropic or thermo-lyotropic, neutral or anionic polysaccharide, and a metal-based nanocomposite consisting of at least one branched cationic polysaccharide wherein metallic nanoparticles are uniformly dispersed and stabilized, characterized in that said three-dimensional nanocomposite materials are obtainable from aqueous solutions of at least one lyotropic, thermotropic or thermo-lyotropic, neutral or anionic polysaccharide, and of a metal-based nanocomposite consisting of at least one branched cationic polysaccharide entrapping the metallic nanoparticles, wherein these aqueous solutions have a ionic strength of at least 50 mM and not higher than 350 mM and a pH of at least of 7 and wherein these aqueous solutions are treated with physical or chemical gelling agents capable to cause the gelation of lyotropic, thermotropic or thermo-lyotropic, neutral or anionic, polysaccharides. These aqueous solutions preferably have an osmolarity comprised in the range from 250 to 300 mM.

Thus, the three-dimensional nanocomposite materials obtainable with such a method of preparing are still an object of the invention.

Furthermore, the three-dimensional nanocomposite materials according to the invention showed to have a strong, broad-spectrum antimicrobial activity and no cytotoxic effects.

Therefore, it is a further object of the invention the use of these three-dimensional nanocomposite materials in biomedical field, in particular for antimicrobial applications. Indeed, the three-dimensional composite materials object of the present invention promise useful applications as biomaterials in both dermatological (e.g. vascular-metabolic cutaneous ulcerations) and orthopedic (e.g. bone prostheses coatings), dental (treatment of periodontal pathogen infections), cardiological, urological (stent coatings) and general surgery therapeutic fields.

### Brief description of the figures

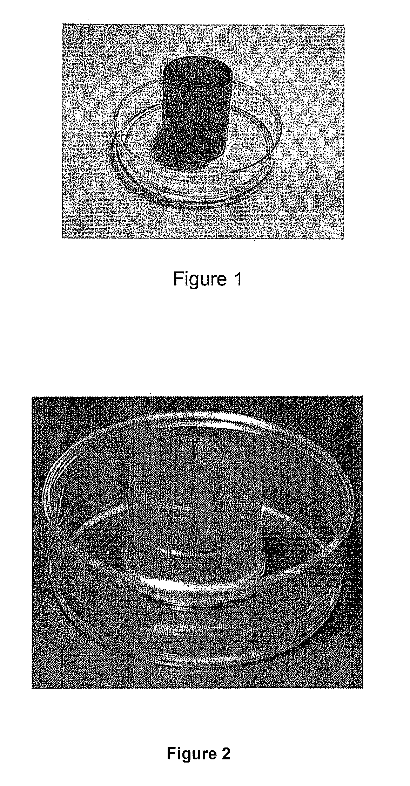

Figure 1 : the figure shows a three-dimensional nanocomposite material in form of hydrogel comprising a metal-based nanocomposite formed by a chitosan derivative with lactose (C: hereinafter referred to as Chitlac: CAS registry number 85941-43-1)-based silver nanoparticles (nAg) in an alginate (A) matrix (AC-nAg gel). The composition is: 0.2% (w/v) Chitlac, 1.5% (w/v) alginate, 0.5 mM AgNO₃, 0.25 mM C₆H₈O₆, 10 mM Hepes buffer, 30 mM CaCO₃, 60 mM GDL (D-glucono-δ-lactone). The preparation of the hydrogel is described in example 8.
Figure 2: the figure shows a three-dimensional nanocomposite material in form of hydrogel comprising a metal-based nanocomposite formed by Chitlac-based gold nanoparticles (nAu) in an Alginate matrix (AC-nAu gel). The composition is: 0.2% (w/V) Chitlac, 1.5% (w/v) alginate, 0.5 mM HAuCl₄, 10 mM Hepes buffer, 30 mM CaCO₃, 60 mM GDL. The preparation is described in example 9.

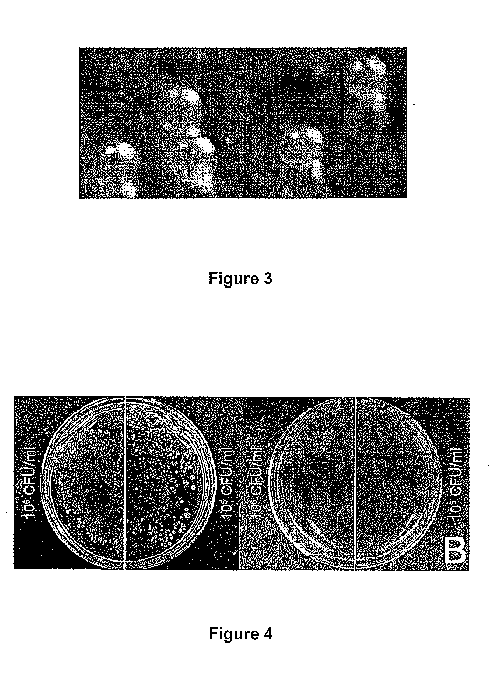

Figure 3: the figure shows three-dimensional nanocomposite material AC-nAg gel microspheres (0.2% (w/v) Chitlac, 1.5% (w/v) alginate, 0.5 mM AgNO₃, 0.25 mM C₆H₈O₆, 10 mM Hepes buffer, 30 mM CaCO₃, 60 mM GDL). The preparation is described in example 13.
Figure 4: the figure shows a bacterial growth test of *E*. *coli* ATCC 25922 on three-dimensional nanocomposite material in form of Alginate-Chitlac hydrogel (AC gel, example 6, on the left) in comparison with a three-dimensional nanocomposite material in form of Alginate-Chitlac-nAg hydrogel (AC-nAg gel, example 8, on the right).

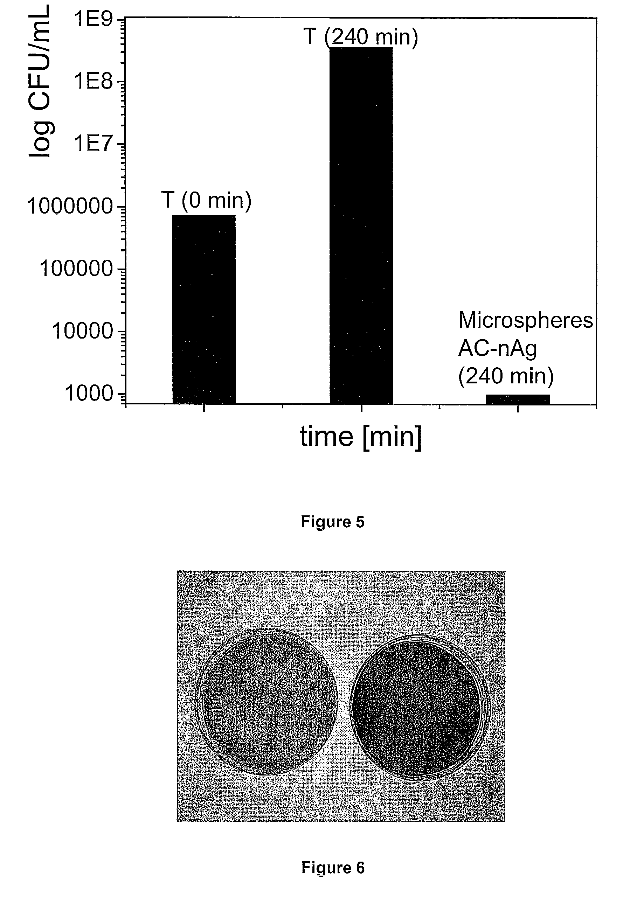

Figure 5: the figure shows a bacterial colony counting test of *E. coli* ATCC 25922 in contact with a suspension containing three-dimensional nanocomposite material AC-nAg microspheres for 240 min. The microsphere preparation is described in example 13. The control is represented by growth in 20% Mueller-Hinton medium (T 0 and 240 min).
Figure 6: the figure shows a bacterial growth test of *E*. *coli* ATCC 25922 on three-dimensional nanocomposite material Alginate-Chitlac hydrogel (AC gel, example 6, on the left) in comparison with a three-dimensional nanocomposite material Alginate-Chitlac-nAu hydrogel (AC-nAg gel, example 9, on the right).

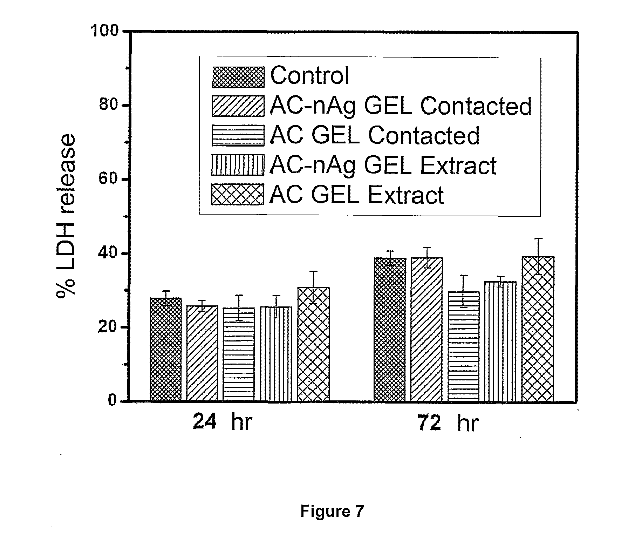

Figure 7: the figure shows a cytotoxicity test on fibroblast cell lines (NIH-3T3), assessed as LDH (lactic dehydrogenase) release, of three-dimensional nanocomposite material AC-nAg gels (example 8, diagonal lines) by contact, and the related extract after 24 and 72 hours in a 37°C medium. The controls are represented by *i)* adherent cells (control, confluent), *ii)* cells in contact with Alginate-Chitlac gel without silver nanoparticles (AC gel, example 6, horizontal lines), *iii)* cells in contact with the liquid culture medium kept in contact with the material for given time (24 hours) (hereafter referred to as "extract") from Alginate-Chitlac gels without silver nanoparticles (AC gel Extract, orthogonal mesh).

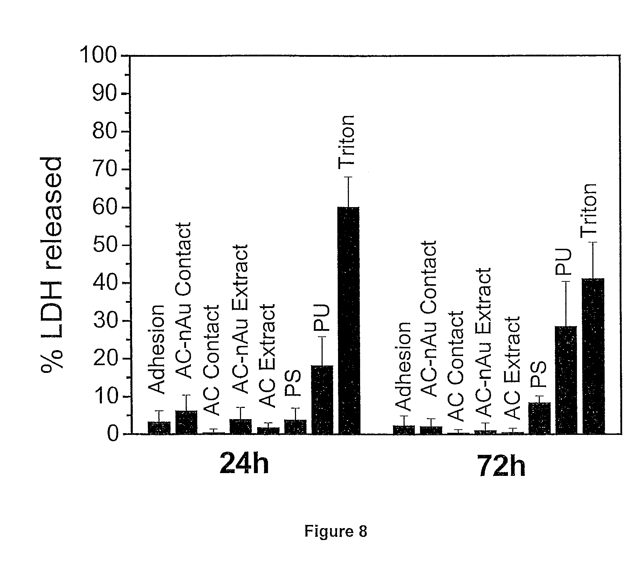

Figure 8: the figure shows a cytotoxicity test (LDH) on osteoblast cell lines (MG63), assessed as LDH (lactic dehydrogenase) release, of three-dimensional nanocomposite material AC-nAu gels (ex. 9, AC-nAu Cont.) by contact, and the related extract (AC-nAu Est.) after 24 and 72 hours in a 37°C medium. The controls are represented by adherent cells (Adhesion), cells in contact with Alginate-Chitlac gels without metal nanoparticles (example 6, AC Cont.), extract from Alginate-Chitlac gels without metal nanoparticles (AC Est), cells in contact with polystyrene disks (PS, negative control of the contacted system), cells in contact with polyurethane disks with ZnDBC (PU, positive control of the contacted system according to ISO 10993-5), cells with a solution of Triton (positive control of the extract).

### Detailed description of the invention

### Definitions

*Three-dimensional structure:* the definition indicates for the purpose of the present application a structure, both hydrated or non-hydrated, capable of maintaining shape and size when not subjected to deformation.

The three-dimensional structure disclosed in the present application are nanocomposite material formed by a neutral or anionic polysaccharides matrix comprising a material formed by metal-nanoparticles uniformly and permanently dispersed in polycationic branched polysaccharides hereinafter described in detail. Thus, in first instance the definition "three-dimensional nanocomposite" is used to indicate the nanocomposite material of the invention.

*Hydrogel:* generally, the term "hydrogel" indicates highly hydrated three-dimensional semisolid structures capable of maintaining shape and size when not subjected to deformation. They may be obtained from semi-dilute solutions of suitably crosslinked polysaccharides.

In the following description "nanocomposite hydrogels" can also be used to indicate, when hydrated, the three-dimensional nanocomposite structures of the invention as previously defined.

*Nanocomposite*: generally, the term "nanocomposite" indicates a system consisting of particles with nanometric size *(fillers)* through a macroscopic material *(matrix).* Being the invention a three-dimensional nanocomposite deriving from the inclusion in a neutral or anionic polysaccharidic matrix of a nanocomposite material (e.g. metallic nanoparticles uniformly and permanently dispersed in branched cationic polysaccharides), this latter material is indicated herein mainly as "metal-based nanocomposite". In particular, the metal-based nanocomposite consists of metallic nanoparticles formed by reduction of metal ions by or in alditolic or aldonic polysaccharidic derivatives of chitosan. Thus, in the following description, besides "metal-based nanocomposite", "metallic-nanoparticles based nanocomposite" is used with reference to this material.

*Colloidal solution (or colloid):* system in which particles with sizes from 1 and 1,000 nm are dispersed in a continuous solvent medium.

*"In situ" gelification:* method of gelling in which there is a controlled release of the gelling agent (e.g., ion Ca²⁺ for alginate). This is achieved by using an inactivated form of the gelling agent (e.g. CaCO₃) which is then released upon adding a second component (e.g. glucono-δ-lactone, GDL).

The objects and advantages of the three-dimensional nanocomposite material described in the present invention will be better understood from the following detailed description where, by the way of non-limiting example of the invention, some examples of preparing three-dimensional nanocomposites and their biological characterization to evaluate the antibacterial activity and cytotoxicity, will be described.

### Description

For the pursued objects, the aspect related to preparation and characterization of a polysaccharides-based nanocomposite system wherein properties related to nano-scale of metallic nanoparticles are exploited, has been addressed. According to the invention three-dimensional nanocomposite materials are formed from a polymeric matrix consisting of a composition of at least one lyotropic, thermotropic or thermo-lyotropic, neutral or anionic, polysaccharide, and a metal-based nanocomposite consisting of at least one branched cationic polysaccharide entrapping metallic nanoparticles uniformly dispersed and stabilized in such a branched cationic polysaccharide. The branched cationic polysaccharides has a double function in the three-dimensional nanocomposite material according of the invention: i) mainly on one side, in properly entrapping and stabilizing the metal nanoparticles and ii) additionally on the other side, in contributing in forming the matrix, which is substantially a composition of polysaccharides (e.g. at least one lyotropic, thermotropic or thermo-lyotropic, neutral or anionic, polysaccharide and at least one branched cationic polysaccharide), inasmuch as the three dimensional matrix is mainly due to the capability of the neutral or anionic polysaccharides to form gels with appropriate gellifying agents. Thus, such a composition of polysaccharides is in a possible embodiment binary and formed by a neutral or acid polysaccharide and a branched cationic polysaccharide.

The neutral or acid polysaccharides useful for the purpose of the invention are: a) acidic polysaccharides, capable of forming lyotropic gels, selected from the group consisting of alginates, pectates, pectinates; b) neutral polysaccharides capable of giving rise to thermotropic gels and, in this case, they are preferably selected from the group consisting of agarose, scleroglucan, schizophyllan, curdlan; c) acidic polysaccharides capable of giving rise to thermo-lyotropic gels and, in this case, are preferably selected from the group consisting of agarose sulphate, I- and κ-carrageenan, cellulose sulphate, gellan gum, rhamsan gum, whelan gum (also addressed to as welan gum), xanthan gum.

The average molecular weight (MW) of neutral or acidic polysaccharides can be up to 2'000 kDa and preferably be from 100 kDa to 1'000 kDa and average molecular weights of 200 kDa are more preferably used.

As well known, these neutral or anionic polysaccharides have the feature of forming three-dimensional structures (gel-like or hydrogels, when hydrated), under suitable conditions. Indeed, the aspect related to hydrogel formation is substantially related to the ability of these neutral or acidic polysaccharides to instantly form hydrogels when contacted with solutions of ions for lyotropic polysaccharides, or with cooled solutions for thermotropic polysaccharides. In the case of acidic thermo-lyotropic polysaccharides, the gelling agents can be both/either physical and/or chemical and thus be either ions or appropriate temperatures or both.

On the contrary, the aspect related to forming and carrying metallic nanoparticles is substantially related to the second polysaccharidic component, that is the branched cationic polysaccharides. For the purposes of the present invention these are alditolic or aldonic branched derivatives of chitosan, wherein the D-Glucosamine units forming the linear chitosan chain bind, by means of the -NH-functional group on carbon atom C2, mono- or oligo-saccharidic alditolic or aldonic polyols residues, equal or different from each other, represented by the general formula (I) 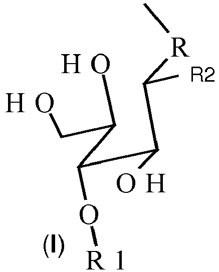 where:
- R is -CH₂- or -CO-;
- R₁ is hydrogen or a monosaccharide, or an oligosaccharide;
- R₂ is -OH or -NHCOCH₃.

For representative purposes, the D-Glucosamine units substituted with mono- or oligo-saccharidic alditolic or aldonic polyols residues in the chitosan branched derivates is represented by the general formula (II), where "n" refers to the overall number of D-Glucosamine units constituting a linear chitosan chain:

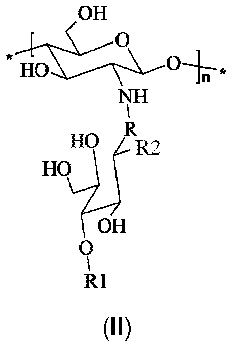

For the purposes of the present invention, in preferred branched derivatives of chitosan, when R₁ is a monosaccharide, said monosaccharide is selected from the group consisting of galactose, glucose, mannose, N-acetyl glucosamine, and N-acetyl galactosamine, and, when R₁ is an oligosaccharide, said oligosaccharide can comprise 2 glycosidic units.

The alditolic or aldonic mono- or oligo-saccharidic residues of general formula (I) preferably are mono- or oligo-saccharides comprising from 1 to 3 glycosidic units and, according to a more preferred aspect, these alditolic or aldonic polyols residues are residues of oligosaccharides comprising from 2 to 3 glycosidic units and yet more preferably are selected from the group of oligosaccharides residues consisting of lactose, cellobiose, cellotriose, maltose, maltotriose, chitobiose, chitotriose, mannobiose as well as from their corresponding aldonic acids. For the purposes of the present invention the most preferred oligosaccharidic derivative of chitosan is the derivative with lactose (Chitlac; CAS registry number 85941-43-1). Furthermore, to uniformly disperse and stabilize the metallic nanoparticles, the chemical substitution degree of chitosan amino groups with these mono- or oligo-saccharides of general formula (I) must be at least 40%. The substitution degree of chitosan amino groups with said mono- or oligo-saccharides is preferably comprised in the range from 50% to 80% and more preferably is 70%.

The average molecular weight (hereinafter referred to as MW) of the chitosan useable for obtaining the mentioned oligosaccharidic derivatives is up to 1'500 kDa and be preferably comprised in the range from 400 kDa to 700 kDa.

The metallic nanoparticles incorporated into the polymeric matrix consisting of these mono-or oligo-saccharidic branched derivates of chitosan are made of metals preferentially selected from silver, gold, platinum, palladium, copper, zinc, nickel and mixtures thereof.

The nanoparticles included into the polymeric matrix consisting of mono- or oligo-saccharidic branched derivates of chitosan have a size in the range from 5 nm to 150, and in particular a controlled average metallic nanoparticle size between 30 and 50 nm.

An essential feature of these nanoparticles is that these nanoparticles are mostly metals in their reduced form, moreover without excluding the residual presence of clusters made of few atoms with an ionic character, and that in their dispersion/stabilization in the polysaccharidic matrix, mono- or oligo-saccharidic side chains close to amino groups of the chitosan itself are involved.

Without being bound to these, the preferred ratios of cationic polysaccharidic matrix and metals are referred to metal-based nanocomposites in the form of colloidal solutions, although said metal-based nanocomposite materials can be also in the form of dehydrated films or powders, and even dialyzed to remove the residual counter-ions from the preparation of the materials themselves. In the metal-based nanocomposites in the form of aqueous colloidal solutions, the ratio of the polysaccharide concentration (expressed as % w/v) over the concentration of the starting metal salt (expressed as molarity) is from 0.0025 to 20 and preferably is 0.2.

Therefore, the metal mass expressed as mg, which may be incorporated per gram of cationic polysaccharide, can be from 3'000 mg/g to 0.3 mg/g and preferentially is 50 mg of metal incorporated per gram of polysaccharide.

Such a component of the three-dimensional nanocomposite materials according to the invention may be prepared under appropriate conditions with aqueous solutions of basic polysaccharides in the presence or absence of exogenous reducing agents.

The method of preparing this component of the three-dimensional nanocomposite materials according to the invention comprises at least:
a) preparing aqueous solutions of branched cationic polysaccharides in a concentration up to 2% (w/v);
b) preparing aqueous solutions of metallic salts in a concentration from 0.1 mM to 20 mM;
c) adding these salt solutions to the solutions of polysaccharides and mixing until obtaining colloidal solutions where metallic nanoparticles are homogeneously dispersed.

A reducing agent is optionally added to the obtained colloidal solutions.

The formation of the metal nanoparticles in the presence of branched cationic polysaccharides produces an exceptionally well-disperse and stabilized metal-nanoparticle system, avoiding the well-known tendency of pre-formed metal nanoparticle to give large agglomerated clusters in solution, which generally leads to loose the benefits related to the nanometric scale.

In its general features, the method of preparing the metal-based nanocomposite is as follows: aqueous solutions of these chitosan branched derivates with mono- or oligo-saccharides are prepared at different concentrations (up to 2% (w/v), preferably in the range from 0.05% (w/v) to 1% (w/v) and more preferably are 0.2%. The polysaccharide solutions are then mixed with solutions of metallic salts chosen from silver, gold, platinum, palladium, copper, zinc, nickel, preferably selected from chlorides, perchlorates and nitrates (e.g. AgNO₃, HAuCl₄, CuSO₄, ZnCl₂, NiCl₂), in order to obtain final concentrations of these metals from 0.1 mM to 20 mM, more preferably from 1 mM to 14 mM, and still more preferably of 1 mM. Appropriate known reducing agents, preferably selected from ascorbic acid, sodium citrate, sodium borohydride and sodium cyanoborohydride, can optionally be added to the solutions in order to obtain metallic-state nanoparticles. The reducing agent is added at concentrations from 0.05 mM to 10 mM and preferably the concentration is 0.5 mM.

However, it was found that otherwise for other polymeric systems, the metal-based nanocomposites formed from chitosan branched derivates with mono- or oligo-saccharides and metallic nanoparticles can be also prepared in the absence of reducing agents, since the side mono- or oligo-saccharidic chains act as reducing agent for metal ions *per se,* and allow to form *in situ* nanoparticles dispersed in the polymeric matrix. In this case, the metallic nanoparticles are obtainable by simply mixing the chitosan derivative solutions with salt solutions of the selected metal at appropriate concentrations. Also in this case, the polysaccharide and metal salt concentrations are as previously reported.

However, in both cases because of the chemical and physical-chemical properties of nitrogen atoms and side substituents existing on the mono- or oligo-saccharidic branched derivative of chitosan, the metal ions interact with macromolecules by means of coordination interactions, while the presence of the side chains of mono-or oligo-saccharides, for example lactose, offers an effective steric hindrance to hamper the natural tendency of nanoparticles to aggregate. The subsequent ion reduction, either caused by an exogenous reducing agent or by the mono- or oligosaccharidic chains of the branched derivatives of chitosan, leads to the formation of nanoparticles stabilized by polysaccharidic chains.

In both cases, the ratio of silver mass which may be incorporated and that of polysaccharide, reported as mg per gram, is as previously reported. Unexpectedly, it was found that mixing in aqueous solutions the polymeric component consisting of the previously mentioned neutral or acidic polysaccharides, and in particular alginates, and the basic polysaccharidic polymeric component, that is branched derivatives of chitosan with the alditolic or aldonic polyols of general formula (I), comprising the metallic nanoparticles, being already a nanocomposite material in nature (e.g. the metal-based nanocomposite), the obtained nanocomposite materials are three-dimensional matrices or stable hydrogels and they are not coacervates in nature, despite the presence of metallic nanoparticles, as well as the nanoparticles remain uniformly dispersed in the branched derivatives of chitosan.

Indeed, hydrogel or three-dimensional matrix formation according of the invention is obtainable by mixing the two components (e.g. neutral or acid polysaccharides and the metal-based nanocomposite) in aqueous solutions having appropriate features by means of a subsequent treatment thereof with suitable agents capable of gelling the anionic or neutral polysaccharide.

In particular, the three-dimensional nanocomposite materials comprising a polymeric matrix consisting of at least one neutral or anionic, lyotropic, thermotropic or thermo-lyotropic polysaccharide, and a metal-based nanocomposite consisting of at least one branched cationic polysaccharide entrapping metallic nanoparticles uniformly dispersed and stabilized, are obtainable from aqueous solutions of the two components having the solutions an ionic strength at least of 50 mM and not higher than 350 mM and a pH at least of 7, and by treating these solutions with chemical or physical gelling agents capable of gelling the neutral or anionic, lyotropic, thermotropic or thermo-lyotropic polysaccharides.

The preferred conditions (substantially concentrations, pH, ionic strength) to obtain the three-dimensional matrices or hydrogels from these two components are typically aqueous solutions having a pH in a physiological range, and in particular between 7 and 8 and more preferably the pH is 7.4, and an osmolarity from 250 to 300 mM, with an ionic strength from 50 mM to 350 mM, and preferably of 150 mM, preferably obtained by adding NaCl at concentrations from 0.05 M to 0.35 M and more preferably of 0.15 M.

The gelling agents may be selected depending of the type of lyotropic anionic polysaccharide from suitable monovalent, bivalent or trivalent ions, and for thermotropic polysaccharides between temperatures not higher than 50°C or not lower than 10°C. As known, for thermo-lyotropic polysaccharides the gelling agents may be both chemical agents, such as ions, and physical agents, such as temperature. The choice between the two types of gelling agents substantially depends as well known in the art on the acidic thermo-lyotropic polysaccharide to be gelled.

For polysaccharides such as alginate and pectate these ions are alkaline-earth ions, excluding magnesium, and transition metals, and preferably selected from the group consisting of calcium, barium, strontium, lead, copper, manganese and mixtures thereof, or they are rare earth ions and preferably selected from the group consisting of gadolinium, terbium, europium and mixtures thereof.

The concentrations of the aqueous solution of these ions adapted for the gelification are higher than 10 mM and preferably from 10 mM to 100 mM and more preferably of 50 mM. The gelling solution preferably contains a concentration of CaCl₂ of 50 mM and an ionic strength of 0.15 M.

In the case of carrageenans, alkaline ions preferably chosen from the group consisting of potassium, rubidium and cesium, at concentrations not lower than 50 mM and preferably from 50 mM to 200 mM and more preferably of 0.1 M, can be used.

In the case of polysaccharidic solutions, which lead to thermotropic hydrogels, such as for example agarose, the hydrogels preparation is performed by cooling below the gel formation temperature. The polysaccharidic solutions are prepared at a temperature above the temperature at which the hydrogel formation by the thermotropic polysaccharide occurs. At this temperature, the thermotropic polysaccharide does not form hydrogels. The temperature at which polysaccharidic solutions are prepared, preferably is in the range from 50°C to 30°C and more preferably is 37°C. The hydrogel formation occurs by dripping the polysaccharidic solution into a gelling bath cooled to a temperature below the gel formation temperature. This temperature preferably is in the range from 10°C to 40 °C and more preferably is 20 °C.

For the purposes of the present invention, the at least binary mixtures of neutral or anionic polysaccharide and of branched basic polysaccharide comprising the metallic nanoparticles have total polymeric concentrations of neutral or anionic polysaccharide up to 4% (w/v). These total polymeric concentrations preferably are in the range from 1.5% to 3% (w/v) and more preferably are 2% (w/v).

For the purposes of the present invention, the weight ratios of acidic polysaccharides and cationic polysaccharides, wherein the metallic nanoparticles are entrapped, are from 8:1 to 1:1 (neutral or anionic polysaccharide : cationic polysaccharide), and preferably from 8:1 to 5:1, and more preferably 7.5:1. Therefore, the three-dimensional nanocomposite materials of the invention are obtainable according to a method of preparing comprising at least the following steps:
i. preparing an aqueous solution of a mixture of at least one neutral or anionic, lyotropic, thermotropic or thermo-lyotropic, polysaccharide and of a metal-based nanocomposite consisting of at least one mono- or oligo-saccharidic derivative of chitosan entrapping metallic nanoparticles (prepared as previously mentioned), said aqueous solution having an ionic strength of at least 50 mM and not higher than 350 mM and at least a pH of 7;
ii. gelling the neutral or anionic, lyotropic, thermotropic or thermo-lyotropic polysaccharide, by treating the aqueous solution of the mixture prepared at the previous step by means of suitable chemical or physical gelling agents.

In particular, this latter step can be realized by dripping through a needle into a solution containing the crosslinking ion for the lyotropic anionic polysaccharides, or into a solution at a suitable temperature for the thermotropic polysaccharides, or by *"in situ"* gelification. For the thermo-lyotropic polysaccharides both ionic solutions and solutions at appropriate temperatures for the gelification process can be employed.

With the above-described method, nanocomposite materials are obtained, in which the polymeric matrix made of polysaccharides is three-dimensional and in the form of a hydrogel or not hydrated if subjected to subsequent dehydration processes. Moreover, these matrices can take various forms as cylinders, microspheres, disks, dried films, powders, or can be extruded to produce fibers.

In case of alginate, the cylinders can be prepared by adding a crosslinking ion in the inactive form, for example CaCO₃ or the Ca-EDTA complex, to the polysaccharidic solution. A slowly hydrolyzing substance is then added, such as for example GDL (D-glucono-δ-lactone). For example, this suspension is transferred within the cylinder-shaped or discoid containers and there kept for 24h. The gel cylinders of the polysaccharidic solutions are then extracted from the containers. The *in situ* formation of cylinders is due to release of calcium ions.

For testing the antimicrobial activity of three-dimensional nanocomposites of the invention, bacterial growth tests on semisolid support and counting test of bacterial colonies in the presence of microspheres of three-dimensional nanocomposites gel are performed; it has been shown that because of the presence of metal nanoparticles, bacteria grow neither on such gel surface nor in a suspension placed in contact with the three-dimensional nanocomposites gel microspheres, thereby underlining a strong antibacterial activity.

Cytotoxicity tests on different eukaryotic cell lines demonstrate that these three-dimensional nanocomposite gels are not cytotoxic even maintaining an effective bactericidal effect.

For illustrative and not limitative purpose, the hydrogel or 3D matrix non hydrated preparation according to the invention as well as the antimicrobial-type biologic activity are described hereinafter.

### Preparation of three-dimensional nanocomposite hydrogels from a solution of one anionic polysaccharide and a metal-based nanocomposite consisting of one oligosaccharidic derivative of chitosan with metallic nanoparticles

### Example 1: synthesis of chitosan derivates with lactose (hereinafter "Chitlac")

Chitosan (1.5 g, acetylation degree 11%) is dissolved into 110 mL of a methanol solution (55 mL) and 1% acetic acid buffer, pH 4.5 (55 mL). 60 mL of a methanol solution (30 mL) and 1% acetic acid buffer, pH 4.5 (30 mL) containing lactose (2.2 g) and sodium cyanoborohydride (900 mg) are added. The mixture is left to stir for 24 hours, transferred to dialysis tubes (*cut off* 12'000Da) and dialyzed against NaCl 0.1 M (2 changes) and against deionized water until a conductivity of 4 µS at 4°C is achieved. Finally, the solution is filtered on Millipore 0.45 µm filters and freeze-dried.

### Example 2: synthesis of chitosan derivates with cellobiose (hereinafter "Chitcell")

Chitosan (1.5g, acetylation degree 11%) is dissolved into 110 mL of a methanol solution (55 mL) and 1% acetic acid buffer, pH 4.5 (55 mL). 60 mL of a methanol solution (30 mL) and 1% acetic acid buffer, pH 4.5 (30 mL) containing cellobiose (2.2 g) and sodium cyanoborohydride (900mg) are added. The mixture is left to stir for 24 hours, transferred to dialysis tubes (*cut off* 12'000Da) and dialyzed against NaCl 0.1 M (2 changes) and against deionized water until a conductivity of 4 µS at 4°C is achieved. Finally, the solution is filtered on Millipore 0.45 µm filters and freeze-dried.

### Example 3: Preparation of metal-based nanocomposite with silver nanoparticles in Chitlac.

Nanoparticles are obtained upon reduction of metal ions with ascorbic acid in Chitlac solutions according to the following procedure: an aqueous Chitlac solution at a concentration of 0.4 % (w/v) is prepared. The Chitlac solutions are then mixed with silver nitrate solutions, so as to obtain a final concentration of AgNO₃ of 1 mM. Then, a solution of ascorbic acid, is added so as to obtain a final concentration of 0.5 mM.

### Example 4: Preparation of metal-based nanocomposite with silver nanoparticles in Chitcel

Nanoparticles are obtained upon reduction of metal ions with ascorbic acid in Chitcel solutions according to the following procedure: an aqueous Chitcel solution at a concentration of 0.4 % (w/v) is prepared. The Chitcel solutions are then mixed with silver nitrate solutions, so as to obtain a final concentration of AgNO₃ of 1 mM. Then, a solution of ascorbic acid is added, so as to obtain a final concentration equal to 0.5 mM.

### Example 5: Preparation of metal-based nanocomposite with gold nanoparticles in Chitlac

Nanoparticles are obtained upon reduction of metal ions by the polysaccharide Chitlac according to the following procedure: an aqueous Chitlac solution at a concentration of 0.4 % (w/V) is prepared. The Chitlac solution is then mixed with a tetrachloroauric acid so as to obtain a final concentration of HAuCl₄ of 1 mM. Example 6: Preparation of cylindrical hydrogels based on alginate-Chitlac and by means of *"in situ"* gelification

To a Chitlac solution, an alginate solution in the presence of NaCl and Hepes buffer is added, so as to obtain the following final concentrations: 1.5% (w/v) alginate, 0.2% (w/v) Chitlac, 0.15 M NaCl, 0.01 M Hepes buffer, pH 7.4. Then, a solution of CaCO₃ (concentration 30 mM) is added and subsequently D-glucono-δ-lactone (GDL) ([GDL]/[Ca²⁺]= 2) is added to allow a slow gelification. For the antibacterial tests 20% Mueller-Hinton medium is added (4.2 g/L). The final solution is transferred to plastic cylinders sized as desired (e.g. 16 mM (0) X 18 mM (h)) and left to gel in the dark for 24 hours.

### Example 7: Preparation of three-dimensional nanocomposite hydrogels based on alginate-Chitlac and metallic nanoparticles by means of "in situ" gelification

A Chitlac solution with metallic nanoparticles prepared according to example 3 is added to an alginate solution (final alginate concentrations up to 4%(w/v) and preferably in the range from 1% (w/v) to 2% (w/v)) in the presence of CaCO₃ (final concentration up to 40 mM and preferably from 15 mM to 30 mM) and then D-glucono-δ-lactone (GDL) ([GDL]/[Ca²⁺]= 2) is added to allow a slow gelification. For the antibacterial tests 20% final Mueller-Hinton medium is added (4.2 g/L). Example 8: Preparation of cylindrical three-dimensional nanocomposite hydrogels based on alginate-Chitlac and silver nanoparticles by means of *"in situ"* gelification To a Chitlac solution with silver nanoparticles prepared according to example 3, an alginate solution in the presence of NaCl and Hepes buffer is added so as to obtain the following final concentrations: 1.5% (w/v) alginate, 0.2% (w/v) Chitlac, 0.5 mM AgNO₃, 0.25 mM C₆H₈O₆, 0.15 M NaCl, 0.01 M Hepes buffer, pH 7.4). Then, a solution of CaCO₃ (concentration 30 mM) is added and subsequently D-glucono-δ-lactone ([GDL]/[Ca²⁺]=2) is added to allow a slow gelification. For the antibacterial tests 20% Mueller-Hinton medium is added (4.2 g/L). The final solution is transferred to plastic cylinders sized as desired (e.g. 16 mM (0) X 18 mM (h)) and left to gel in the dark for 24 hours.

It is worth underlining that particle aggregation or polymeric phase separation is absent both during and after the gelification. As seen in Figure 1, the obtained three-dimensional nanocomposite material is a yellow-orange hydrogel (the color varies depending on silver concentration).

### Example 9: Preparation of cylindrical three-dimensional hydrogels based on alginate-Chitlac and gold nanoparticles by means of "in situ" gelification

To a Chitlac solution with gold nanoparticles prepared according to example 5, an alginate solution in the presence of NaCl and Hepes buffer is added, so as to obtain the following final concentrations: 1.5% (w/v) alginate, 0.2% (w/v) Chitlac, 0.5 mM HAuCl₄, 0.15M NaCl, 0.01 M Hepes buffer, pH 7.4). Then, a solution of CaCO₃ (concentration 30 mM) is added and subsequently D-glucono-δ-lactone (GDL) ([GDL]/[Ca²⁺]=2) is added to allow a slow gelification. For the antibacterial tests 20% Mueller-Hinton medium is added (4.2 g/L). The final solution is transferred to plastic cylinders sized as desired (e.g. 16 mM (0) X 18 mM (h)) and left to gel in the dark for 24 hours.

An obtained three-dimensional nanocomposite hydrogel sample is shown in figure 2.

### Example 10: preparation of three-dimensional nanocomposite spheres by means of a syringe from alginate-Chitlac solutions (in mannitol) with silver nanoparticles.

A polysaccharidic solution with the following final concentrations was prepared. 1.5% (w/v) alginate, 0.2% (w/v) Chitlac, 0.5 mM AgNO₃, 0.25 mM C₆H₈O₆, 0.15 M NaCl, 0.01 M Hepes buffer, pH 7.4. The solution was dripped using a syringe provided with a 23G needle, into a solution containing 50 mM CaCl₂ and 0.15 M mannitol, 10 mM Hepes buffer (pH 7.4) under stirring by means of a magnetic rod. The spheres were kept under agitation in the gelling bath for 10 min before being removed and washed with deionized water.

### Example 11: preparation of three-dimensional nanocomposite spheres by means of a syringe from alginate-Chitlac solutions (in NaCl) with silver nanoparticles

A polysaccharidic solution with the following final concentrations was prepared. 1.5% (w/v) alginate, 0.2% (w/v) Chitlac, 0.5 mM AgNO₃, 0.25 mM C₆H₈O₆, 0.15 M NaCl, 0.01 M Hepes buffer, pH 7.4. The solution was dripped using a syringe provided with a 23G needle, into a solution containing 50 mM CaCl₂ and 0.15 M mannitol, 10 mM Hepes buffer (pH 7.4) under stirring by means of a magnetic rod. The spheres were kept under agitation in the gelling bath for 10 min before being removed and washed with deionized water.

### Example 12: preparation of three-dimensional nanocomposite spheres by means of a syringe from alginate-Chitlac solutions with gold nanoparticles.

A polysaccharidic solution with the following final concentrations was prepared. 1.5% (w/v) alginate, 0.2% (w/v) Chitlac, 0.5 mM HAuCl₄, 0.15 M NaCl, 0.01 M Hepes buffer, pH 7.4. The solution was dripped using a syringe provided with a 23G needle, into a solution containing 50 mM CaCl₂ and 0.15 M mannitol (gelling bath) under stirring by means of a magnetic rod. The spheres were kept under agitation in the gelling bath for 10 min before being removed and washed with deionized water.

### Example 13: preparation of three-dimensional nanocomposite microspheres by means of an electrostatic bead generator from alginate-Chitlac solutions with silver nanoparticles.

A polysaccharidic solution with the following final concentrations was prepared. 1.5% (w/v) alginate, 0.2% (w/v) Chitlac, 0.5 mM AgNO₃, 0.25 mM C₆H₈O₆, 0.15 M NaCl, 0.01 M Hepes buffer, pH 7.4. The solution was dripped into a gelling bath containing 50 mM CaCl₂ and 0.15 nm mannitol under stirring by means of a magnetic rod. The microsphere size is controlled by using an electrostatic generator, which allows to act on the surface tension of the drops so as to reduce the size thereof. The conditions employed typically were: voltage 5kV, internal needle diameter 0.7 mM, distance between gelling bath and needle 4 cm, binary polymeric solution flow rate 10 mL/min. The microspheres were left in the gelling solution under stirring for 10 min before being removed and washed with deionized water.

Obtained three-dimensional nanocomposite microsphere samples are shown in figure 3.

### Example 14: preparation of three-dimensional nanocomposite microspheres by means of an electrostatic bead generator from alginate-Chitlac solutions with gold nanoparticles

A polysaccharidic solution with the following final concentrations was prepared: 1.5% (w/v) alginate, 0.2% (w/v) Chitlac, 0.5 mM HAuCl₄, 0.15M NaCl, 0.01 M Hepes buffer, pH 7.4. The solution was dripped into a gelling bath containing 50 mM CaCl₂ and 0.15 nm mannitol under stirring by means of a magnetic rod.

The microsphere size is controlled by using an electrostatic bead generator, which allows to act on the surface tension of the drops so as to reduce the size thereof. The conditions employed typically are: voltage 5 kV, internal needle diameter 0.7 mM, distance between gelling bath and needle 4 cm, binary polymeric solution flow rate 10 mL/min. The microspheres are left in the gelling solution under stirring for 10 min before being removed and washed with deionized water.

### Example 15: Preparation of three-dimensional nanocomposite hydrogels with high surface/volume ratio based on alginate-Chitlac and silver nanoparticles by means of "in situ" gelification.

To a Chitlac solution with silver nanoparticles prepared according to example 3, an alginate solution in the presence of NaCl and Hepes buffer is added so as to obtain the following final concentrations: 1.5% (w/v) alginate, 0.2% (w/v) Chitlac, 0.5 mM AgNO₃, 0.25 mM CₑH₈O₆, 0.15 M NaCl, 0.01 M Hepes buffer, pH 7.4. Then, a solution of CaCO₃ (concentration 30 mM) is added and subsequently D-glucono-δ-lactone (GDL) ([GDL]/[Ca²⁺]=2 is added to allow a slow gelification. For the antibacterial tests 20% final Mueller-Hinton medium is added (4.2 g/L). The final solution is poured onto smooth surfaces (slides, Petri dishes, etc.) and left to gel in the dark for 24 hours.

### Example 16: Preparation of three-dimensional nanocomposite dehydrated films based on alginate-Chitlac and silver nanoparticles by means of "in situ" gelification.

To a Chitlac solution with silver nanoparticles prepared according to example 3, an alginate solution in the presence of NaCl and Hepes buffer is added so as to obtain the following final concentrations: 1.5% (w/v) alginate, 0.2% (w/v) Chitlac, 0.5 mM AgNO₃, 0.25 mM C₆H₈O₆, 0.15 M NaCl, 0.01 M Hepes buffer, pH 7.4. Then, a solution of CaCO₃ (concentration 30 mM) is added and subsequently D-glucono-δ-lactone (GDL) ([GDL]/[Ca²⁺]=2) is added to allow a slow gelification. For the antibacterial tests 20% final Mueller-Hinton medium is added (4.2 g/L). The final solution is poured onto smooth surfaces (slides, Petri dishes, etc.) and left to gel in the dark for 24 hours. Then, the three-dimensional nanocomposite gel is air-dried so as to obtain a three-dimensional nanocomposite solid dehydrated film.

### Example 17: Preparation of three-dimensional nanocomposite hydrogels with high surface/volume ratio based on alginate-Chitlac and gold nanoparticles by means of "in situ" gelification

To a Chitlac solution with gold nanoparticles prepared according to example 5, an alginate solution in the presence of NaCl and Hepes buffer is added so as to obtain the following final concentrations: 1.5% (w/v) alginate, 0.2% (w/v) Chitlac, 0.5 mM HAuCl₄, 0.15M NaCl, 0.01 M Hepes buffer, pH 7.4. Then, a solution of CaCO₃ (concentration 30 mM) is added and subsequently D-glucono-δ-lactone (GDL) ([GDL]/[Ca²⁺]=2) is added to allow a slow gelification. For the antibacterial tests 20% final Mueller-Hinton medium is added (4.2 g/L). The final solution is poured on smooth surfaces (slides, Petri dishes, etc.) and left to gel in the dark for 24 hours.

### Example 18: Preparation of three-dimensional nanocomposite films based on alginate-Chitlac and gold nanoparticles by means of "in situ" gelification

To a Chitlac solution with gold nanoparticles prepared according to example 5, an alginate solution in the presence of NaCl and Hepes buffer is added so as to obtain the following final concentrations: 1.5% (w/v) alginate, 0.2% (w/v) Chitlac, 0.5 mM HAuCl₄, 0.15M NaCl, 0.01 M Hepes buffer, pH 7.4. Then, a solution of CaCO₃ (concentration 30 mM) is added and subsequently D-glucono-δ-lactone (GDL) ([GDL]/[Ca²⁺]=2) is added to allow a slow gelification. For the antibacterial tests 20% final Mueller-Hinton medium is added (4.2 g/L). The final solution is poured on smooth surfaces (slides, Petri dishes, etc.) and left to gel in the dark for 24 hours. Then, the three-dimensional nanocomposite gel is air-dried so as to obtain a three-dimensional nanocomposite solid film.

### Example 19: Preparation of microspheres

The microspheres of the above-reported specific examples 13 and 14 were prepared according to known methods and in particular by: a) using syringes, with which of the solutions of one anionic polysaccharide and one metal-based nanocomposite mono-or oligo-saccharidic derivative of chitosan with metallic nanoparticles are manually dripped into a suitable gelling bath, b) using one Electrostatic Bead Generator, developed by Prof. Gudmund Skjåk-Bræk of Institute of Biotechnology of NTNU University of Trondheim (Norway) and described by Strand et al., 2002, J. of Microencapsulation 19, 615-630. Such an apparatus consists of an electrostatic bead generator with an adjustable voltage (up to 10kV) by means of a suitable switch, connected to an autoclavable needle support contained in a safety stand made of Plexiglas.

By means of a system outside the stand, consisting of a syringe adjusted by a pump and connected to a pipe made of lattice having an internal diameter of 1 mm, the starting solution is dripped into a crystallizator (within the stand) containing the gelling solution, in which an electrode is inserted. The instrument generates a constant potential difference between the needle tip and the free surface of the gelling solution, which may be adjusted and ranges from 0 to 10 kV. The potential difference causes the sudden detachment of the polymer drop (negatively charged) from the needle tip and thus allows to have capsules even with small sizes (<200 µm). The capsule sizes may be adjusted by even varying other factors, such as the internal needle diameter, the distance of the needle from the gelling solution surface, the polymer flow rate.

### Example 20: Preparation of cylinders

The gel cylinders and disks of the above-reported specific examples 6, 8 and 9 were prepared by pouring the solution containing the polysaccharides and the metal-based nanocomposite into cylinder-shaped containers. The cylindrical hydrogel size depends on the size of the latter ones. The cylinder-shaped container dimensions typically are 18 mm in height and 16 mm in internal diameter, while those of discoid containers are 8 mm in height and 16 mm in internal diameter, even if different sizes (height and internal diameter) are fully allowable.

### Example 21: Biological characterizations

### A. Antibacterial activity

For testing the antibacterial activity of three-dimensional nanocomposite gels, different bacterial strains at various concentrations were smeared on the gel surfaces. Both Gram negative strains *(Escherichia coli, Pseudomonas aeruginosa)* and Gram positive strains *(Staphylococcus aureus, Staphylococcus epidermidis)* were tested. The controls are represented by agar gels and alginate-Chitlac gels without nanoparticles (AC gels). After "*overnight*" incubation the bacterial colonies are clearly evident on controls but completely absent on gels containing nanoparticles (AC-nAg gels) (figure 4), thus proving the bactericidal action efficacy performed by these materials.

Moreover, nanocomposite gel microspheres containing silver nanoparticles (AC-nAg) are made and contacted with bacterial solutions *(Escherichia coli*); the control is represented by alginate-Chitlac gel microspheres (without silver nanoparticles). The results demonstrate that the concentration of bacterial colonies increases in the control but decreases by more than three logarithmic units in the case of microspheres with silver nanoparticles.

Even the nanocomposite microspheres obtained according to example 13 and nanocomposite hydrogels obtained according to examples 6 and 9 proved to be able to exert an effective antimicrobial action, as shown in figures 5 and 6, respectively.

### B. Cytotoxicity

Tests to evaluate gel cytotoxicity on eukaryotic cell lines, such as osteoblasts (MG63), hepatocytes (HEPG2) and fibroblasts (3T3), were performed. In the test, the release of lactate dehydrogenase enzyme (LDH) by cells is measured assessed, which is related to cellular damage and death. The LDH tests demonstrate that these three-dimensional nanocomposite gels do not cause cytotoxic effects to the tested cells, as it can be seen in figure 7, for example in the case of fibroblasts. Indeed, after 24 and 72 hours no significant difference in the release of lactate dehydrogenase (LDH) between the cells treated with AC-nAg and the controls (i.e. cells treated with alginate-Chitlac gels and non-treated cells) are found.

Comparable results are reported in the case of similar three-dimensional nanocomposite alginate-Chitlac-based gels containing gold nanoparticles (AC-nAu gels); as seen in figure 8, this type of gel is not cytotoxic for the eukaryotic cells as well.

The combination of these results allows to conclude that the three-dimensional nanocomposite according to the invention, such as alginate-Chitlac-based hydrogels containing nanoparticles formed and stabilized in Chitlac, are provided with a homogeneous structure in which the nanoparticles do not aggregate and have a strong bactericidal activity, without being toxic for the eukaryotic cells. These new three-dimensional nanocomposite systems provide the following advantages:
- the development of an integrated system which combines the ability of branched derivatives of chitosan of forming and stabilizing metallic nanoparticles with a bactericidal activity, with the already demonstrated biological properties in cellular stimulation by the oligosaccharidic side-chains of such chitosan derivatives (Marsich et al., "Alginate/lactose-modified chitosan hydrogels: A bioactive biomaterial for chondrocyte encapsulation", Journal of biomedical materials research-Part A, 2008 Feb; 84(2):364-76). This new "Glyco-Nanotechnological" approach will provide new instruments for designing materials, which exploit the bioactivity provided by the "glycobiological" component and the special properties of the nanoparticles;
- the possibility of preparing three-dimensional gels with a mixed composition of *i)* neutral or anionic polysaccharides and *ii)* cationic branched mono-or oligo-saccharidic derivates of chitosan entrapping metallic nanoparticles, by exploiting the total miscibility under appropriate conditions between two polymers separately belonging to each of the two classes *i)* and *ii),* although they have opposite charges (for the polyanions of class *i*) and despite the presence of metallic nanoparticles. On the other hand, as known, coacervation phenomena with anionic polysaccharide, and in particular with alginate, occur by using other non-oligosaccharide-branched cationic polymers such as chitosan;
- the obtained nanocomposite gels are found not to be toxic to eukaryotic cells even if they have a strong bactericidal activity;
- the used method of gelification allows to obtain materials with shapes and sizes which may be suitable for the different application needs (for example wet and dry films, *scaffolds,* microspheres, fibers, etc.).

## Claims

1. A three-dimensional nanocomposite material comprising a polymeric matrix consisting of at least one lyotropic, thermotropic or thermo-lyotropic, neutral or anionic polysaccharide, and a metal-based nanocomposite consisting of at least one cationic branched polysaccharide uniformly entrapping metallic nanoparticles, wherein the neutral or anionic polysaccharide is gelled by means of suitable chemical or physical gelling agents.

2. The three-dimensional nanocomposite material according to claim 1, wherein the cationic branched polysaccharides are branched derivatives of chitosan, wherein the D-Glucosamine units forming the chitosan linear chain bind, by means the functional group-NH- on carbon atom C2, alditolic or aldonic polyols residues, equal or different from each other, represented by the general formula (II) 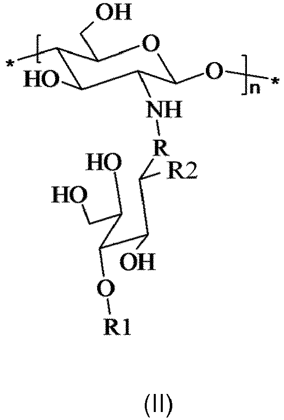 where:
- R is -CH₂- or -CO-;
- R₁ is hydrogen, a monosaccharide, or an oligosaccharide;
- R₂ is -OH or -NHCOCH₃.

3. The three-dimensional nanocomposite material according to claim 2, wherein the alditolic or aldonic polyols residues are residues of mono- or oligosaccharides comprising from 1 to 3 glycosidic units.

4. The three-dimensional nanocomposite material according to claim 2, wherein, when R₁ is a monosaccharide, said monosaccharide is selected from the group consisting of galactose, glucose, mannose, N-acetyl glucosamine, and N-acetyl galactosamine.

5. The three-dimensional nanocomposite material according to claim 2, wherein the alditolic or aldonic polyols residues are selected from the group consisting of residues of lactose, cellobiose, cellotriose, maltose, maltotriose, chitobiose, chitotriose, mannobiose and aldonic acids thereof.

6. The three-dimensional nanocomposite material according to claim 2, wherein the branched derivatives of chitosan have a chemical substitution degree of the D-Glucosamine unit amine group higher than 40% and up to 80%.

7. The three-dimensional nanocomposite material according to claim 1, wherein the metallic nanoparticles comprised in the metal-based nanocomposite with cationic branched polysaccharides are of metals selected from the group consisting of silver, gold, platinum, palladium, copper, zinc, nickel and mixtures thereof.

8. The three-dimensional nanocomposite material according to claim 1, wherein the nanoparticles have an average size comprised in the range from 5 nm to 150 nm.

9. The three-dimensional nanocomposite material according to claim 1, wherein the metal mass in mg per g of cationic polysaccharide is comprised in the range from 3'000 mg/g to 0.3 mg/g.

10. The three-dimensional nanocomposite material according to claim 1, wherein the weight ratios of neutral or anionic polysaccharides to cationic branched polysaccharides comprising the metallic nanoparticles are comprised in the range from 8:1 to 1:1 (neutral or anionic polysaccharides:cationic polysaccharides).

11. The three-dimensional nanocomposite materials according to one of the claims 1-10, wherein the polymeric matrices are hydrogels or non-hydrated matrices.

12. The three-dimensional nanocomposite materials according to one of the claims 1-11, wherein the polymeric matrices are in different forms selected from cylinder shaped, microspheres, *scaffolds,* gel-slabs, films and fibers.

13. A three-dimensional nanocomposite material according to one of the claims 1-12 for use as an antimicrobial agent.

14. The three-dimensional nanocomposite material according to claim 13 for use in wound healing.

15. The three-dimensional nanocomposite material according to claim 13 for use in surgical applications.

16. The three-dimensional nanocomposite material according to claim 13 for use in agriculture and food applications.

17. A method for preparing three-dimensional nanocomposite materials according to claims 1-12, **characterized by** the fact that said three-dimensional nanocomposite materials are obtainable by preparing aqueous solutions of mixtures of at least one lyotropic, thermotropic or thermo-lyotropic, neutral or anionic polysaccharide and of a metal-based nanocomposite consisting of at least one cationic branched polysaccharide uniformly entrapping metallic nanoparticles, wherein said aqueous solutions have an ionic strength of at least 50 mM and not higher than 350 mM and a pH of at least 7, and by treating said aqueous solutions with chemical or physical agents capable of gelling the lyotropic, thermotropic or thermo-lyotropic, neutral or anionic, polysaccharides.

18. The method for preparing three-dimensional nanocomposite materials according to claim 17, wherein the aqueous solutions have an osmolarity comprised in the range from 250 to 300mM.

19. The method for preparing three-dimensional nanocomposite materials according to claim 17, wherein the gelling agents for the lyotropic or thermo-lyotropic polysaccharides are aqueous solutions of salts of ions selected from monovalent, divalent or trivalent ions having concentrations are from 10 mM and up to 100mM.

20. The method for preparing three-dimensional nanocomposite materials according to claim 17, wherein the gelling agents for the thermotropic or thermo-lyotropic polysaccharides have temperatures comprised in the range from 10°C to 40°C.

21. The method for preparing three-dimensional nanocomposite materials according to claim 17, wherein the polymeric concentrations of neutral or anionic polysaccharides are higher than 1.5% and up to 4% (w/v).

## Patentansprüche

1. Dreidimensionales Nanokompositmaterial das eine Polymermatrix aufweist, bestehend aus mindestens einem lyotropischen, thermotropischen oder thermolyotropischen, neutralen oder anioschen Polysaccharid und einem metallbasierten Nanokomposit bestehend aus mindestens einem kationisch verzweigten Polysaccharid mit gleichmäßig eingeschlossenen metallischen Nanopartikeln, wobei das neutrale oder anionische Polysaccharid durch geeignete chemische oder physikalische Geliermittel geliert ist.

2. Dreidimensionales Nanokompositmaterial gemäß Anspruch 1, wobei die kationisch verzweigten Polysaccharide verzweigte Chitosanderivate sind, in denen die D-Glucosamin-Einheiten die lineare Chitosan-Verkettung bilden, durch die funktionale NH-Gruppe auf dem Kohlenstoffatom C2, alditolischen oder aldonischen Polyolrückständen gleicher oder unterschiedlicher Art, dargestellt durch die allgemeine Formel (II): 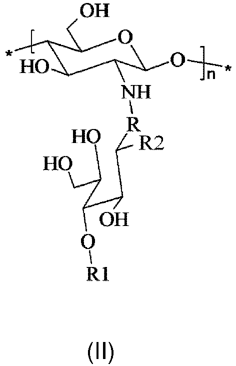 wobei:
- R -CH2- oder -CO- ist
- R1 Wasserstoff, ein Monosaccharid oder ein Oligosaccharid ist
- R2 -OH oder -NHCOCH3 ist.

3. Dreidimensionales Nanokompositmaterial gemäß Anspruch 2, wobei die alditolischen oder aldonischen Polyolrückstände Rückstände von Mono- oder Oligosacchariden sind, die 1 bis 3 Glykosidgruppen aufweisen.

4. Dreidimensionales Nanokompositmaterial gemäß Anspruch 2, wobei, für den Fall, dass R1 ein Monosaccharid ist, das Monosaccharid aus der Gruppe von Galaktose, Glukose, Mannose, N-Acetyl-Glukosamin und N-Acetyl-Galaktosamin gewählt wird.

5. Dreidimensionales Nanokompositmaterial gemäß Anspruch 2, wobei die alditolischen oder aldonischen Polyolrückstände aus der Gruppe von Rückständen von Laktose, Cellobiose, Cellotriose, Maltose, Maltotriose, Chitobiose, Chitotriose, Mannobiose und aldonischen Säuren davon gewählt wird.

6. Dreidimensionales Nanokompositmaterial gemäß Anspruch 2, wobei die verzweigten Chitosanderivate einen chemischen Substitutionsgrad der D-Glukosamineinheit der Amingruppe zwischen 40% und 80% aufweist.

7. Dreidimensionales Nanokompositmaterial gemäß Anspruch 1, wobei die metallischen Nanopartikel in dem metallbasierten Nanokomposit mit kationisch verzweigten Polysacchariden aus der Gruppe der Metalle aufweisend Silber, Gold, Platin, Palladium, Kupfer, Zink, Nickel und Mischungen daraus gewählt werden.

8. Dreidimensionales Nanokompositmaterial gemäß Anspruch 1, wobei die Nanopartikel eine durchschnittliche Größe zwischen 5 nm und 150 nm haben.

9. Dreidimensionales Nanokompositmaterial gemäß Anspruch 1, wobei die Metallmasse in mg pro Gramm des kationischen Polysaccharids im Bereich zwischen 3000 mg/g und 0,3 mg/g liegt.

10. Dreidimensionales Nanokompositmaterial gemäß Anspruch 1, wobei das Gewichtsverhältnis von neutralen oder anionischen Polysacchariden zu kationisch verzweigten Polysacchariden, die die metallischen Nanopartikel enthalten, im Bereich von 8:1 bis 1:1 liegt (neutrale oder anionische Polysaccharide : kationische Polysaccharide)

11. Dreidimensionale Nanokompositmaterialien gemäß einem der Ansprüche 1 bis 10, wobei die Polymermatritzen Hydrogels oder nicht-hydratisierte Matritzen sind.

12. Dreidimensionale Nanokompositmaterialien gemäß einem der Ansprüche 1 bis 11, wobei die Polymermatritzen in verschiedenen Formen, wie zylinderförmig, Mikrokugeln, gerüstförmig, Gelscheiben, Filmen oder Fasern vorliegen.

13. Dreidimensionale Nanokompositmaterialien gemäß einem der Ansprüche 1 bis 12 zur Verwendung als Antimikrobiotikum.

14. Dreidimensionale Nanokompositmaterialien gemäß Anspruch 13 zur Verwendung bei der Wundheilung.

15. Dreidimensionale Nanokompositmaterialien gemäß Anspruch 13 zur Verwendung bei chirurgischen Anwendungen.

16. Dreidimensionale Nanokompositmaterialien gemäß Anspruch 13 zur Verwendung bei Anwendungen in Landwirtschaft und bei Nahrungsmitteln.

17. Verfahren zur Herstellung dreidimensionaler Nanokompositmaterialien gemäß den Ansprüchen 1 bis 12, **dadurch gekennzeichnet, dass** die dreidimensionalen Nanokompositmaterialien erhalten werden durch Herstellung von wässrigen Lösungen aus Mischungen von mindestens einem lyotropischen, thermotropischen oder thermolyotropischen, neutralen oder anioschen Polysaccharid und einem metallbasierten Nanokomposit, bestehend aus mindestens einem kationisch verzweigten Polysaccharid mit gleichmäßig eingeschlossenen metallischen Nanopartikeln, wobei die wässrigen Lösungen eine Ionenstärke zwischen 50 mM und 350 mM und einen pH-Wert von mindestens 7 haben und durch Behandlung der wässrigen Lösungen mit chemischen oder physikalischen Mitteln sind die lyotropischen, thermotropischen oder thermolyotropischen, neutralen oder anionischen Polysaccharide gelierbar.

18. Verfahren zur Herstellung dreidimensionaler Nanokompositmaterialien gemäß Anspruch 17, wobei die wässrigen Lösungen eine Osmolarität zwischen 250 und 300 mM haben.

19. Verfahren zur Herstellung dreidimensionaler Nanokompositmaterialien gemäß Anspruch 17, wobei die Geliermittel für die lyotropischen oder thermolyotropischen Polysaccharide wässrige Lösungen aus einwertigen, zweiwertigen oder dreiwertigen Ionensalzen mit Konzentrationen zwischen 10 mM und 100 mM sind.

20. Verfahren zur Herstellung dreidimensionaler Nanokompositmaterialien gemäß Anspruch 17, wobei die Geliermittel für die thermotropischen oder thermolyotropischen Polysaccharide eine Temperatur zwischen 10 °C und 40 °C haben.

21. Verfahren zur Herstellung dreidimensionaler Nanokompositmaterialien gemäß Anspruch 17, wobei die Polymerkonzentration der neutralen oder anionischen Polysaccharide zwischen 1,5% und 4% liegt (Gew. / Vol).

## Revendications

1. Matériau nanocomposite tridimensionnel comprenant une matrice polymère constituée d'au moins un polysaccharide lyotrope, thermotrope ou thermolyotrope, neutre ou anionique, et un nanocomposite à base de métal constitué d'au moins un polysaccharide ramifié cationique piégeant uniformément des nanoparticules métalliques, dans lequel le polysaccharide neutre ou anionique est gélifié par des agents gélifiants chimiques ou physiques convenables.

2. Matériau nanocomposite tridimensionnel selon la revendication 1, dans lequel les polysaccharides ramifiés cationiques sont des dérivés ramifiés de chitosan, dans lequel les motifs D-glucosamine formant la chaîne linéaire du chitosan lient, par le groupe fonctionnel -NH- sur l'atome de carbone C2, des résidus de polyols alditoliques ou aldoniques, identiques ou différents les uns des autres, représentés par la formule générale (II) 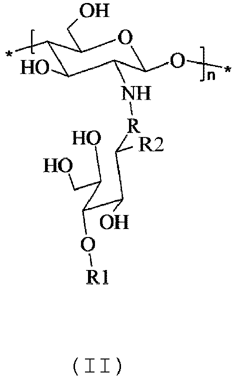 où :
- R est -CH₂- ou -CO- ;
- R₁ est un atome d'hydrogène, un monosaccharide ou un oligosaccharide ;
- R₂ est -OH ou -NHCOCH₃.

3. Matériau nanocomposite tridimensionnel selon la revendication 2, dans lequel les résidus de polyols alditoliques ou aldoniques sont des résidus de mono- ou d'oligo-saccharides comprenant de 1 à 3 motifs glycosidiques.

4. Matériau nanocomposite tridimensionnel selon la revendication 2, dans lequel, lorsque R₁ est un monosaccharide, ledit monosaccharide est choisi dans le groupe constitué du galactose, du glucose, du mannose, de la N-acétylglucosamine et de la N-acétylgalactosamine.

5. Matériau nanocomposite tridimensionnel selon la revendication 2, dans lequel les résidus de polyols alditoliques ou aldoniques sont choisis dans le groupe constitué des résidus de lactose, cellobiose, cellotriose, maltose, maltotriose, chitobiose, chitotriose, mannobiose et les acides aldoniques de ceux-ci.

6. Matériau nanocomposite tridimensionnel selon la revendication 2, dans lequel les dérivés ramifiés de chitosan ont un degré de substitution chimique du groupe amine du motif D-glucosamine supérieur à 40 % et allant jusqu'à 80 %.

7. Matériau nanocomposite tridimensionnel selon la revendication 1, dans lequel les nanoparticules métalliques comprises dans le nanocomposite à base d'un métal avec les polysaccharides ramifiés cationiques sont des métaux choisis dans le groupe constitué de l'argent, de l'or, du platine, du palladium, du cuivre, du zinc, du nickel et des mélanges de ceux-ci.

8. Matériau nanocomposite tridimensionnel selon la revendication 1, dans lequel les nanoparticules ont une taille moyenne comprise dans la plage de 5 nm à 150 nm.

9. Matériau nanocomposite tridimensionnel selon la revendication 1, dans lequel la masse de métal en mg par g de polysaccharide cationique est comprise dans la plage de 3 000 mg/g à 0,3 mg/g.

10. Matériau nanocomposite tridimensionnel selon la revendication 1, dans lequel les rapports pondéraux des polysaccharides neutres ou anioniques aux polysaccharides cationiques ramifiés comprenant les nanoparticules métalliques sont compris dans la plage de 8:1 à 1:1 (polysaccharides neutres ou anioniques : polysaccharides cationiques).

11. Matériaux nanocomposites tridimensionnels selon l'une des revendications 1 à 10, dans lesquels les matrices polymères sont des hydrogels ou des matrices non hydratées.

12. Matériaux nanocomposites tridimensionnels selon l'une des revendications 1 à 11, dans lesquels les matrices polymères ont des formes différentes choisies parmi les cylindres, les microsphères, les squelettes, les plaques de gel, les films et les fibres.

13. Matériau nanocomposite tridimensionnel selon l'une des revendications 1 à 12 pour une utilisation comme agent antimicrobien.

14. Matériau nanocomposite tridimensionnel selon la revendication 13 pour une utilisation dans la cicatrisation d'une plaie.

15. Matériau nanocomposite tridimensionnel selon la revendication 13 pour une utilisation dans des applications chirurgicales.

16. Matériau nanocomposite tridimensionnel selon la revendication 13 pour une utilisation dans des applications agricoles et alimentaires.

17. Procédé de préparation de matériaux nanocomposites tridimensionnels selon les revendications 1 à 12, **caractérisé par le fait que** lesdits matériaux nanocomposites tridimensionnels sont susceptibles d'être obtenus par préparation de solutions aqueuses de mélanges d'au moins un polysaccharide lyotrope, thermotrope ou thermo-lyotrope, neutre ou anionique et d'un nanocomposite à base de métal constitué d'au moins un polysaccharide ramifié cationique piégeant uniformément des nanoparticules métalliques, dans lequel lesdites solutions aqueuses ont une force ionique d'au moins 50 mM et non supérieure à 350 mM et un pH d'au moins 7, et par traitement desdites solutions aqueuses avec des agents chimiques ou physiques capables de gélifier les polysaccharides lyotropes, thermotropes ou thermolyotropes, neutres ou anioniques.

18. Procédé de préparation de matériaux nanocomposites tridimensionnels selon la revendication 17, dans lequel les solutions aqueuses ont une osmolarité comprise dans la plage de 250 à 300 mM.

19. Procédé de préparation de matériaux nanocomposites tridimensionnels selon la revendication 17, dans lequel les agents gélifiants pour les polysaccharides lyotropes ou thermo-lyotropes sont des solutions aqueuses de sels d'ions choisis parmi les ions monovalents, divalents ou trivalents ayant des concentrations de 10 mM et allant jusqu'à 100 mM.

20. Procédé de préparation de matériaux nanocomposites tridimensionnels selon la revendication 17, dans lequel les agents gélifiants pour les polysaccharides thermotropes ou thermo-lyotropes ont des températures comprises dans la plage de 10 °C à 40 °C.

21. Procédé de préparation de matériaux nanocomposites tridimensionnels selon la revendication 17, dans lequel les concentrations polymères des polysaccharides neutres ou anioniques sont supérieures à 1,5 % et vont jusqu'à 4 % (pd/vol).
